# EUROPÄISCHE PATENTANMELDUNG

(11) **EP 0 576 888 A2**
(43) Veröffentlichungstag der Anmeldung: **05.01.1994**
(21) Anmeldenummer: 93109339.7
(22) Anmeldetag: 11.06.1993
(51) Int. Cl.: C07C 33/03, C07C 29/38, C07C 29/32

(54) **Verfahren zur Herstellung von (S)- und (R)-But-3-en-2-ol und deren Derivaten aus L- bzw. D-Milchsäureestern**

(30) Priorität: 13.06.1992 DE 4219510
(71) Anmelder: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Klingler, Franz Dietrich, Dr., D-6103 Griesheim (DE); Psiorz, Manfred, Dr., D-6507 Ingelheim (DE)

(57) **Zusammenfassung**

Die vorliegende Erfindung betrifft ein neues Verfahren zur Herstellung von (S)- sowie (R)-But-3-en-2-ol (1-Buten-3-ol bzw. Methylvinylcarbinol) und deren Derivaten der allgemeinen Formel (1).

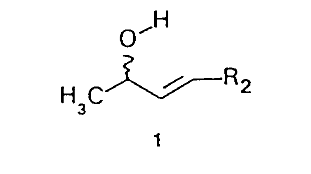

## Beschreibung

Die vorliegende Erfindung betrifft ein neues Verfahren zur Herstellung von (S)- sowie (R)-But-3-en-2-ol (1-Buten-3-ol bzw. Methylvinylcarbinol) und deren Derivaten der allgemeinen Formel 1 a bzw. 1 b, 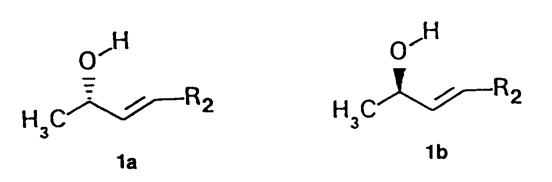
worin R₂ Wasserstoff und C1-C6-Alkyl bedeuten kann, ausgehend von L- beziehungsweise D-Milchsäureestern.

C1-C6-Alkyl steht im allgemeinen für einen verzweigten oder unverzweigten Kohlenwasserstoffrest mit 1 bis 6 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
Methyl, Ethyl, Propyl, 1-Methylethyl (Isopropyl), Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbuty, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie z.B. Methyl, Ethyl, Propyl, Isopropyl, Butyl und tert.-Butyl.

Enantiomerenreinen Butenolen kommt als Synthons für die Herstellung enantiomerer Folgeprodukte - insbesondere für die Synthese von pharmazeutischen Wirkstoffen - 1-Buten-3-ole eine große Bedeutung zu.

Verfahren zur Herstellung der einzelnen Stereoisomeren des Methylvinylcarbinols sind aus dem Stand der Technik bekannt.

So beschreiben J. Kenyon et al. [J. Kenyon und D.R. Snellgrove, J. Chem. Soc., 1926, 1169] ein Verfahren, das es ermöglicht, ausgehend von einem Enantiomerengemisch des Methylvinylcarbinols über dessen Phthalsäurehalbester und deren anschließende Racematspaltung mittels Brucin sowie Verseifung des gewünschten Stereoisomeren des Phthalsäurehalbesters S(+)-But-3-en-2-ol zugänglich zu machen. Der Nachteil dieser Reaktionsfolge besteht im wesentlichen darin, daß zahlreiche Kristallisationsschritte zu durchlaufen sind.

Daneben beschreiben T. Ibrahim et al. [T. Ibrahim, T.J. Grattan und J.S. Whitehurst, J. Chem. Soc. Perkin Trans I, 1990, 3317] ein Verfahren ausgehend von 3-Chlorbutan-2-on, welches mittels Hefe zunächst zu den entsprechenden (2S, 3S)- und (2S,3R)-3-Chlorbutan-2-olen reduziert wird. Die nachfolgende Veretherung mit 2-Methoxypropen sowie Dehydrohalogenierung mit Kalium-tert.-butylat führt schließlich zum angestrebten (S)( + )-But-3-en-2-ol.

Nachteilig an dieser Reaktionsfolge ist, daß die Reduktion mit Hefe lediglich in sehr hoher Verdünnung durchgeführt werden kann und somit die Herstellung einer verhältnismäßig kleinen Menge des Butenols ein relativ großes Reaktorvolumen erfordern.

Des weiteren beschreiben T. Yoshida et al. [T. Yoshida, M. Kaneoya, M. Uchida und H. Morita, Japanische Offenlegungsschrift 1-132 399 vom 24. Mai 1989] ein Verfahren zur sog. optischen Spaltung von (R,S)-2-Buten-3-ol, bei dem nach Umsetzung mit Tributyrin und nachfolgende enzymatische Behandlung die beiden stereoisomeren Butenole zugänglich gemacht werden können. Dieses Verfahren leidet jedoch unter einer schlechten Ausbeute und liefert Produkte mit schlechten Charakteristika.

Weiterhin beschreiben G. Giacomelli et al. [G. Giacomelli, A.M. Caporusso und L. Lardicci, Tetrahedron Lett. 22 (1981) 3663] die asymmetrische Reduktion von 1-Buten-3-on unter Verwendung optisch aktiver Trialkylalane. Bei der dort beschriebenen Reaktion kann das (R)-1-Buten-3-ol jedoch nur in einem Enantiomerenüberschuß (e.e.) von lediglich 7% erhalten werden.

Einen höheren Enantiomerenüberschuß liefert eine von H.C. Brown et al. [H.C. Brown und G.G. Pai, J. Org. Chem. 50 (1985) 1384] beschriebene Reaktion, bei der 1-Buten-3-on mit sog. Alpine-Borane@ (B-(3-Pinanyl)-9-borabicyclo[3.3.1 ]nonan) stereoselektiv reduziert wird. Auf diesem Wege ist beispielsweise das (R)-1-Buten-3-ol nach einer Reaktionszeit von 5 Tagen in einem Enantiomerenüberschuß (e.e.) von 60% jedoch nur in einer Ausbeute von 30% zugänglich.

Ziel der vorliegenden Erfindung ist es, (R)- bzw. (S)-3-Buten-2-ole - und deren Derivate - in guten Ausbeuten und in einem hohen Enantiomerenüberschuß verfügbar zu machen sowie ein Verfahren zu deren Herstellung zu entwickeln, das die Nachteile der aus dem Stand der Technik bekannten Verfahren umgeht.

Diese Aufgaben werden durch das nachfolgend beschriebene und in den Beispielen ausgeführte Verfahren ausgehend von kommerziell erhältlichen D-oder L-Milchsäurealkylestern, wobei die entsprechenden Alkylester und besonders die Ethylester bevorzugt werden.

Das erfindungsgemäße Verfahren besteht aus vier Reaktionsschritten:
Auf der ersten Reaktionsstufe wird die freie Hydroxylgruppe - beispielsweise des L-Milchsäureethylesters (2) geschützt. Der Schutz erfolgt mit den an sich aus dem Stand der Technik bekannten Schutzgruppen, die gegen Reduktionsmittel resistent sind [T.W. Greene und P.G.M. Wuts, Protective Groups in Organic Synthesis, 2^{nd} Edition, John Wiley & Sons, Inc., New York, 1991, S. 10 ff.]. Bevorzugt findet die Tetrahydropyranyl-Schutzgruppe Verwendung. Dabei erfolgt der Schutz der Hydroxylgruppe nach den ebenfalls aus dem Stand der Technik an sich bekannten Methoden. In einer bevorzugten Ausführungsform wird die Hydroxylgruppe des Milchsäureethylesters in Substanz mit 3,4-Dihydro-2H-pyran in Anwesenheit einer katalytischen Menge einer Säure in einem Temperaturbereich von -10 bis + 50 _{°} C verethert. 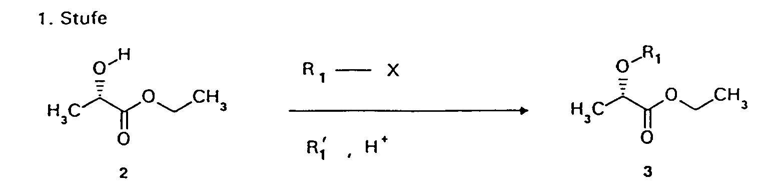
   Die Reaktion kann jedoch auch in Gegenwart eines Lösungsmittels durchgeführt werden, wobei sich als Solvenzien alle Lösungsmittel eignen, die keinen nachteiligen Einfluß auf den Reaktionsverlauf haben. Beispielsweise seien halogenierte Kohlenwasserstoffe - wie Dichlormethan - genannt.

Bevorzugt wird als Säure eine Mineralsäure eingesetzt - worunter Salzsäure besonders bevorzugt wird. Dabei wird die Reaktion in einem Temperaturbereich von 0 bis 40 _{°} C durchgeführt wird.

Anschließend wird das Reaktionsgemisch fraktioniert destilliert, wobei es zweckmäßig ist, ein gegebenenfalls vorhandenes Lösungsmittel vorher abzudestillieren und der so geschützte Milchsäureester isoliert.

Auf der Reaktionsstufe 2 erfolgt die Reduktion des im ersten Reaktionsschritt hergestellten Milchsäureethylesters zum entsprechenden Propanal-Derivat (4), welches ebenfalls nach den aus dem Stand der Technik bekannten Verfahren durchgeführt werden kann [R.C. Larock, Comprehensive Organic Transformations - A Guide to Functional Group Preparations, VCH-Publishers, Weinheim 1989, S. 621 und zit. Lit.]. Bevorzugt wird die Reduktion mit ggf. komplexen - Aluminiumhydriden durchgeführt, worunter Organoaluminiumhydride - wie z.B. Dialkylaluminiumhydride, wie Diisobutylaluminiumhydrid (DIBAH) oder Alkalialkoxyaluminiumhydride, auch komplexe Alkali-Organoaluminiumhydride, wie Lithium-tris-(tert.-butoxy)-aluminiumhydrid oder Natrium-bis-(methoxy-ethoxy)aluminiumhydrid (Vitride@) - bevorzugt werden. Besonders bevorzugt wird DIBAH als Reduktionsmittel in Form einer 35 %-igen Lösung in Toluol eingesetzt. 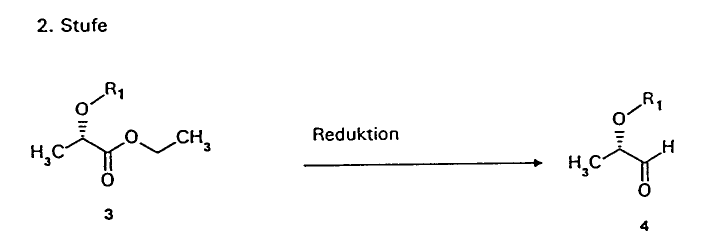
Die Reduktion wird bevorzugt in einem Lösungsmittel durchgeführt. Als Reaktionsmedien eignen sich alle inerten Lösungsmittel, die keinen nachteiligen Einfluß auf die Reduktionsreaktion ausüben.

Hierzu gehören insbesondere aliphatische oder aromatische Kohlenwasserstoffe, wie z.B. Petrolether, Benzol, Toluol, Xylol, worunter Toluol besonders bevorzugt wird. Die Reduktion wird bei Temperaturen von unterhalb 0 _{°} C durchgeführt, bevorzugt in einem Bereich von -50 bis -20 °C und besonders bevorzugt bei -40 _{°} C.

Zur Aufarbeitung wird das Reaktionsgemisch zunächst der Hydrolyse unterworfen, wobei Mischungen aus niederen aliphatischen Alkoholen - wie z.B. Methanol, Ethanol, Propanol, iso-Propanol - und Wasser bevorzugt werden. Besonders bevorzugt wird eine Mischung aus 100 Volumenteilen Wasser und ca. 37 Volumenteilen Methanol bei einer Temperatur von -40 _{°} C. Bei der Hydrolyse liegt die Temperatur ebenfalls in den oben angegebenen Bereichen. Nach erfolgter Hydrolyse läßt man das Reaktionsgemisch auf Raumtemperatur (ca. 20 °C) erwärmen, wobei das bei der Hydrolyse entstandene Aluminiumhydroxyd als dickflüssiger Brei ausfällt. Das Aluminiumhydroxyd wird abgetrennt und das Filtrat im Vakuum eingeengt der verbliebene Rückstand unter vermindertem Druck fraktioniert destilliert und der so erhaltene resultierende Aldehyd (4) isoliert.

Auf der dritten Reaktionsstufe erfolgt die Umwandlung des Aldehyds (4) in das gewünschte Alken im Rahmen einer Wittig-Reaktion [C. Ferri Reaktionen der organischen Synthese, Georg Thieme Verlag, Stuttgart, 1978, S. 354 und zit. Lit.; J. March, Advanced Organic Chemistry, J. Wiley & Sons, New York, 1985, S. 845 und zit. Lit.]. 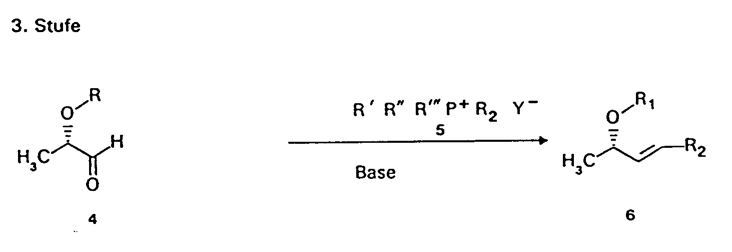
Die Herstellung der gewünschten Phosphoniumsalze vom Typ (5) - R₂ = C₁-C₆-Alkyl - ist ebenfalls aus dem Stand der Technik bekannt. Bevorzugt werden Alkyltriphenylphosphoniumhalogenide als sog. Wittig-Reagenzien eingesetzt. Zur Herstellung des Alkens wird der Aldehyd zu einer Suspension bzw. Lösung des Phosphoniumsalzes und einer Base gegeben.

Als Reaktionsmedien eignen sich alle Lösungsmittel, die den Reaktionsverlauf nicht nachteilig beeinflussen, worunter Ether - wie z.B. Di-n-butylether, Glykoldimethylether, Diglykoldimethylether, Tetrahydrofuran - oder Sulfoxide - wie beispielsweise Dimethylsulfoxid oder Tetrahydrothiophen-1,1-dioxid (Sulfolan). Ebenso ist es möglich, Mischungen der genannten Lösungsmittel einzusetzen.

Dabei wird besonders Tetrahydrofuran als Reaktionsmedium bevorzugt. Als Basen werden Alkaliorganyle oder Alkoxyalkaliverbindungen eingesetzt, worunter Lithiumalkyle bevorzugt werden. Besonders bevorzugt wird n-Butyllithium als 15 %-ige Lösung in n-Hexan eingesetzt.

Die Reaktionstemperatur kann - in Abhängigkeit von dem jeweils eingesetzten Reaktionsmedium - in einem weiten Bereich variiert werden und wird nach unten durch eine zu geringe Reaktionsgeschwindigkeit und nach oben durch ein Überhandnehmen von Nebenreaktionen (beispielsweise die Zersetzung des Tetrahydrofurans durch die als Basen eingesetzten Lithiumalkyle) eingegrenzt.

Dabei hat es sich als vorteilhaft erwiesen, das Phosphoniumsalz und die Base bei einer Temperatur im Bereich von 0 bis 40 °C, zu vereinigen wobei der Bereich von 20 bis 25 °C besonders bevorzugt wird.

Die Zugabe der Carbonylverbindung (4) kann ebenfalls innerhalb eines verhältnismäßig großen Temperaturintervalls erfolgen, wobei es sich als vorteilhaft erwiesen hat, die Reaktionslösung während der Zugabe zu kühlen. Bevorzugt wird eine Temperatur in einem Intervall von 0 bis 40 °C, worin der Bereich von 15-20 °C besonders bevorzugt wird.

Nach erfolgter Umsetzung wird das ausgefallene Phosphinoxid - bzw. Triphenylphosphinoxid aus dem Reaktionsgemisch - beispielsweise auf dem Wege der Filtration - abgetrennt, das Filtrat unter vermindertem Druck eingeengt und der verbliebene Rückstand mit Wasser verrührt. Danach wird die resultierende wässerige Suspension mit einem organischen Extraktionsmittel extrahiert. Als Extraktionsmittel eignen sich zweckmäßigerweise Lösungsmittel, die mit Wasser nicht mischbar sind, worunter im vorliegenden Fall aliphatische oder aromatische Kohlenwasserstoffe wie verzweigte oder unverzweigte Alkane, Benzol, Toluol oder Xylol bevorzugt werden. - Besonders bevorzugt wird n-Hexan als Extraktionsmittel eingesetzt.

Die organische Extraktionslösung wird - ggf. unter Zuhilfenahme eines Trockenmittels vom Wasser befreit und nach dem Abtrennen des Trockenmittels - unter vermindertem Druck eingeengt und der Rückstand - ebenfalls - unter vermindertem Druck fraktioniert destilliert und das daraus resultierende Alken vom Typ (6) isoliert.

Da die Wittig-Reaktion in einer sehr großen Zahl von Varianten durchführbar ist, wird hierdurch - je nach Art der ausgewählten Variante - die Wahl anderer Reaktionsmedien, Basen sowie anderer Reaktionsbedingungen ermöglicht.

Auf der vierten und letzten Reaktionsstufe erfolgt das Abspalten der Schutzgruppe. Die einzelnen Verfahren zum Abspalten der verschiedenen Schutzgruppen sind ebenfalls aus dem Stand der Technik bekannt. [T.W. Greene und P.G.M. Wuts, Protective Groups in Organic Synthesis, 2^{nd} Edition, J. Wiley & Sons., Inc. New York, 1991, S. 10 ff]. Nach dem erfindungsgemäßen Verfahren wird das auf Stufe 3 erhaltene Alken (6) in einem geeigneten Lösungsmittel gelöst. Als Lösungsmittel eignen sich - insbesondere für das Abspalten der Tetrahydropyranyl-Schutzgruppe - organische Säuren - wie z.B. Essigsäure - oder Alkohole - wie z.B. Methanol oder Ethanol - oder Polyalkohole - wie z.B. Ethylenglykol oder Glycerin -, worunter Ethylenglykol besonders bevorzugt wird. 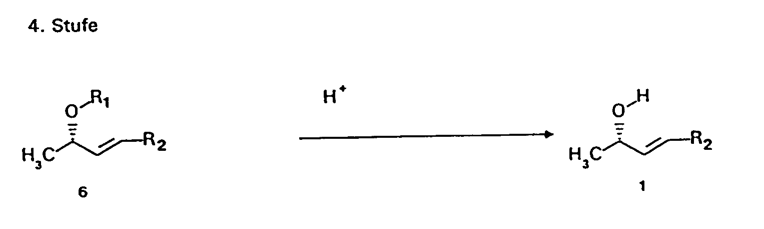
Die Abspaltung erfolgt dabei in Gegenwart einer organischen Säure, worunter organische Sulfonsäuren bevorzugt sind. Besonders bevorzugt wird p-Toluolsulfonsäure eingesetzt.

Die Reaktionstemperatur kann - in Abhängigkeit von dem jeweils eingesetzten Reaktionsmedium - in einem weiten Bereich variiert werden und wird nach unten lediglich durch eine zu geringe Reaktionsgeschwindigkeit und nach oben durch das Überhandnehmen von Nebenreaktionen eingegrenzt.

Bevorzugt wird die Abspaltung der Schutzgruppe bei einer Temperatur im Bereich von 20 bis 40 _{°} C und - besonders bevorzugt - in einem Intervall von 20 bis 30°C und ganz besonders bevorzugt in einem Bereich von 20 bis 25 _{°} C durchgeführt.

Das Isolieren des nunmehr ungeschützten Alkenols vom Typ (1) kann auf verschiedene Arten erfolgen, die dem Fachmann geläufig sind. Sofern es die chemischen und physikalischen Eigenschaften des Reaktionsproduktes erlauben, kann es - unter vermindertem Druck - aus der Reaktionsmischung abdestilliert werden. Bei Verwendung von Lösungsmitteln, die im Vergleich zum Alkenol (1) einen höheren Siedepunkt aufweisen, ist es auch denkbar, zuerst das Reaktionsmedium abzudestillieren und dann das Reaktionsprodukt - ggf. fraktioniert - zu destillieren.

Das hier und in den nachfolgenden Beispielen beschriebene Verfahren liefert beispielsweise das S(+)-But-3-en-2-ol in einer optischen Reinheit von 98.9 %.

Die eingangs genannten Aufgaben werden durch die in den Beispielen 1 - 8 beschriebenen Verfahren gelöst. Verschiedenartige, andere Ausgestaltungen des Verfahrens werden für den Fachmann aus der vorliegenden Beschreibung ersichtlich. Es wird jedoch ausdrücklich darauf hingewiesen, daß die Beispiele und die diesen zugeordnete Beschreibung lediglich zum Zweck der Erläuterung und Beschreibung vorgesehen und nicht als Einschränkung der Erfindung anzusehen sind. Insbesondere wird darauf hingewiesen, daß die in den Beispielen zur Herstellung von (S)-But-3-en-2-ol beschriebene Synthesefolge auf die Herstellung des entsprechenden R-Enantiomeren übertragbar ist.

Ebenso kann als Ausgangsmaterial jeder andere beliebige L- bzw. D-Milchsäurealkylester eingesetzt werden.

### Beispiel

### Herstellung von S(+)-But-3-en-2-ol

### 1. Stufe:

In einer 1.5 I Sulfierapparatur werden 608,2 g (97 %-ig, 5.0 Mol) L-Milchsäureethylester vorgelegt und unter Rühren innerhalb von 15 Minuten 433,0 g (5.0 Mol) 3.4-Dihydro-2H-pyran zugegeben. Die Temperatur sinkt dabei von 25°C auf 20°C ab. Danach kühlt man die Lösung im Eis/Kochsalz-Bad auf 0°C ab und tropft 4 ml einer etherischen Salzsäurelösung in 2 Minuten zu. Die Temperatur steigt dabei langsam auf 12°C an. Nach 30 minütigem Rühren wird das Kältebad entfernt, wobei innerhalb von 30 Minuten die Temperatur auf 40°C ansteigt. Die Lösung wird 20 Stunden lang bei 20-25°C gerührt. Anschließend wird die Rekationslösung über eine verspiegelte Füllkörperkolonne fraktioniert destilliert.

Ausbeute: 882 g farbloses Öl (84 % d. Th.) Kp_{0.4} x10²pa: 91 ⁰ C

### 2. Stufe

In einer mit Stickstoff gespülten 4 I Sulfierapparatur werden 1200 ml absolutes Toluol vorgelegt und unter Rühren 160 g (0.8 Mol) des in Reaktionsstufe 1 hergestellten Tetrahydropyranylethers zugegeben. Danach kühlt man die Lösung mit einem Trockeneis/Acetonbad auf eine Temperatur von -40 °C ab und gibt innerhalb 60 Minuten bei -40°C 407 g (1.0 Mol) Diisobutylaluminiumhydrid - in Form einer 35 %-igen Lösung in Toluol - zu. Anschließend wird 2 Stunden lang bei -40 °C nachgerührt. Mit einer Lösung von 100 ml destilliertem Wasser und 36.7 ml Methanol wird die Reaktionslösung innerhalb eines Zeitraumes von einer Stunde bei einer Temperatur von -40 _{°} C hydrolysiert. Danach entfernt man das Kältebad und läßt die Reaktionsmischung auf Raumtemperatur erwärmen. Dabei fällt Aluminiumhydroxid in Form eines dicken Breis aus. Man läßt 1 Stunde bei einer Temperatur von 25°C nachrühren. Das ausgefallene Aluminiumhydroxid wird über ein Seitz-K 400 Filter, welches mit Celite-Filtererde belegt ist, abgesaugt und mit 3 x 300 ml Toluol gewaschen. Das Filtrat wird anschließend bei 40 °C und einem Druck von 53.3 x 10²Pa eingeengt und der Rückstand im Vakuum fraktioniert destilliert. Ausbeute: 70 g farbloses Öl (55.3 % d. Th.) ^{Kp}₂₆.₆ x ₁₀2_{Pa}: 95-97 °C.

### 3. Stufe

In einer mit N₂ gespülten 6 I Sulfierapparatur werden 2400 ml absolutes Tetrahydrofuran vorgelegt und unter Rühren 174 g (0.48 Mol) Triphenylmethylphosphoniumbromid zugegeben. Die Suspension wird bei Raumtemperatur innerhalb einer Stunde mit 264 ml (0.53 Mol) einer 15 %-igen Lösung von Butyllithium in Hexan versetzt. Dabei löst sich die Suspension auf, wobei eine gelbe Lösung entsteht. Im Anschluß daran wird 1 Stunde bei einer Temperatur von 20-25°C nachgerührt. Danach wird eine Lösung von 70 g (0.44 Mol) des aus Reaktionsstufe 2 resultierenden Aldehyds in 600 ml Tetrahydrofuran bei einer Temperatur im Bereich von 15-20_{°}C (Eiskühlung) innerhalb eines Zeitraumes von 90 Minuten dem Wittig-Reagenz zugetropft. Dabei entfärbt sich die Lösung und das entstehende Triphenylphosphinoxid fällt in Form von farblosen Kristallen aus. Es wird 2 Stunden bei einer Temperatur von 20-25 °C nachgerührt. Das ausgefallene Triphenylphosphinoxid wird über ein Druckfilter, welches mit Seitz-Filter K 400 belegt ist, abfiltriert. Das Filtrat wird im Vakuum eingeengt und der Rückstand mit 1 Liter Wasser verrührt. Danach Extrahiert man die Suspension 3 x mit je 500 ml n-Hexan. Die vereinigten Extrakte mit wasserfreiem Natriumsulfat werden getrocknet und nach dem Abfiltrieren des Trockenmittels bei einem Druck von 20 kPa und 40 °C eingeengt. Der Rückstand wird im Vakuum fraktioniert destilliert (Kühlertemperatur min. 0°C). Ausbeute: 34 g farbloses Öl (49.6 % d. Th.) Kp_{26.6x10}2_{Pa}: 70-71 ° C

### 4. Stufe

In einem 250 ml Dreihalskolben werden 34 g (0.22 Mol) des in Reaktionsstufe 3 isolierten Butenderivates 110 ml Ethylenglykol gelöst und 4,2 g (22 mMol) p-Toluolsulfonsäure zugegeben. Nach 2 Stunden starkem Rühren bei 20-25°C wird im Wasserstrahlvakuum bei einer Badtemperatur von 85°C und unter einem Druck von 35 Torr (46.1 x 10²Pa) das S(+)But-3en-2-ol aus der Reaktionslösung abdestilliert. Dabei wird der Kühler mit Kühlflüssigkeit von -5°C beschickt. Es werden 14 g Rohprodukt isoliert, welches im Wasserstrahlvakuum über eine Dornenrohrkolonne destilliert wird.
Ausbeute: 10,2 g farbloses Öl (64,4 % d. Th.) Kp 80 x 10² Pa 38-40 °C [a] = D20 + 31,5 ° pur d = 0,832
opt. Reinheit: 98,9 % S(+)But-3en-2-ol 1,1 % R(-)But-3en-2-ol

## Patentansprüche

1. Verfahren zur Herstellung von enantiomerenreinen (S)- oder (R)-But-3-en-2-ol bzw. Derivaten der allgemeinen Formel (1) fallen 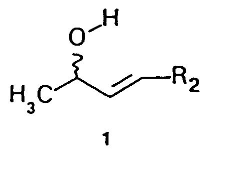
worin
R₂ Wasserstoff oder einen verzweigten oder unverzweigten Kohlenwasserstoffrest mit 1 bis 6 C-Atomen, der gegebenenfalls mit einem oder mehreren Halogenatom(en) substituiert sein kann,
bedeuten kann, dadurch gekennzeichnet, daß man
1. die Hydroxylgruppe des (S)- bzw. (R)-Milchsäurealkylesters (2) mit einer Verbindung Rᵢ-X umsetzt, in welcher X eine durch Sauerstoff substituierbare Austrittsgruppe und R₁ eine gegenüber Reduktionsmitteln resistente Schutzgruppe verkörpert, oder mit einer Verbindung R₁' umsetzt, die auf der Basis einer Additionsreaktion befähigt ist das Butenol unter Ausbildung einer Etherfunktion zu addieren und den so geschützten Milchsäureester (3) isoliert 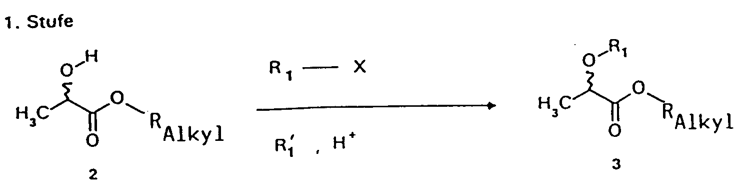
und
2. den geschützten Milchsäureethylester (3) mit einem Reduktionsmittel zu dem entsprechenden Propanal (4) in Gegenwart eines Lösungsmittels umsetzt und den resultierenden Aldehyd (4) isoliert 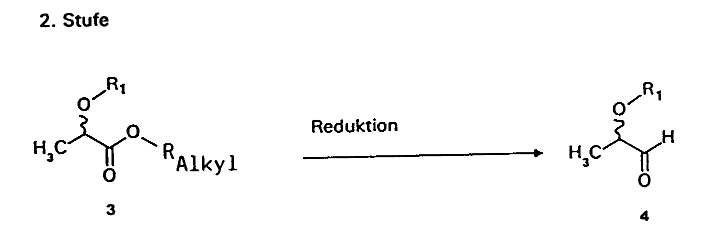
und
3. den Aldehyd (4) mit einem Wittig-Reagenz der allgemeinen Formel (5) in Gegenwart einer Base umsetzt und das geschützte Alkenol (6) isoliert 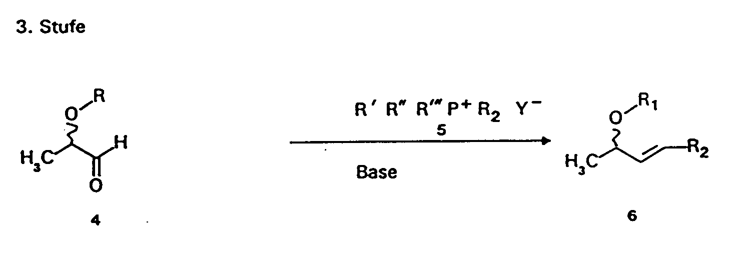
und
4. die Schutzgruppe R₂ vom Alkenolderivat (6) abspaltet und das resultierende (S)- bzw. (R)-But-3-en-2-ol der allgemeinen Formel (1) isoliert.

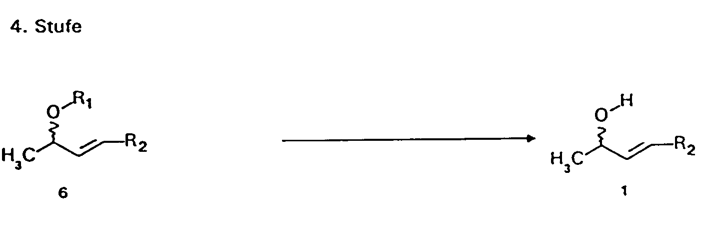

2. Verfahren nach Anspruch 1, dadurch gekennzeichnet, daß man im Teilschritt 1 zum Schutz der Hydroxylgruppe den (S)- bzw. (R)-Milchsäureethylester mit 3,4-Dihydro-2H-pyran in Gegenwart einer Säure bei einer Temperatur in einem Bereich von -10 bis + 50 _{°} C umsetzt.

3. Verfahren nach Anspruch 2, dadurch gekennzeichnet, daß man als Säure eine Mineralsäure einsetzt und die Reaktion bei einer Temperatur im Bereich von 0 bis 40 _{°} C durchführt.

4. Verfahren nach Anspruch 3, dadurch gekennzeichnet, daß man als Mineralsäure Salzsäure einsetzt.

5. Verfahren nach Anspruch 1, dadurch gekennzeichnet, daß man in Teilschritt 2 als Reduktionsmittel ein komplexes Alkali-Aluminiumhydrid einsetzt.

6. Verfahren nach Anspruch 1, dadurch gekennzeichnet, daß man ein Organoaluminiumhydrid einsetzt.

7. Verfahren nach Anspruch 6, dadurch gekennzeichnet, daß man als Organoaluminiumhydrid ein Dialkylaluminiumhydrid einsetzt.

8. Verfahren nach Anspruch 7, dadurch gekennzeichnet, daß man als Dialkylaluminiumhydrid Diisobutylaluminiumhydrid (DIBAH) - in einer 35 %-igen Lösung in Toluol - verwendet.

9. Verfahren nach Anspruch 1, dadurch gekennzeichnet, daß man in Teilschritt 2 als Lösungsmittel einen aliphatischen oder aromatischen Kohlenwasserstoff einsetzt.

10. Verfahren nach Anspruch 9, dadurch gekennzeichnet, daß man Toluol als Lösungsmittel einsetzt.

11. Verfahren nach Anspruch 1, dadurch gekennzeichnet, daß man die Reduktion in einem Temperaturintervall von -50 bis -20 _{°} C durchführt.

12. Verfahren nach Anspruch 11, dadurch gekennzeichnet, daß man die Reduktion bei einer Temperatur von -40 °C durchführt.

13. Verfahren nach Anspruch 1, dadurch gekennzeichnet, daß man in Teilschritt 3 als Wittig-Reagenz ein C1-C6-Triarylalkylphosphoniumhalogenid einsetzt.

14. Verfahren nach Anspruch 13, dadurch gekennzeichnet, daß man als Wittig-Reagenz Triphenylmethylphosphoniumiodid einsetzt.

15. Verfahren nach Anspruch 1, dadurch gekennzeichnet, daß man in Teilschritt 3 als Base ein Alkalialkyl oder eine Alkoxyalkaliverbindung einsetzt.

16. Verfahren nach Anspruch 15, dadurch gekennzeichnet, daß man als Alkalialkyl eine lithiumorganische Verbindung einsetzt.

17. Verfahren nach Anspruch 16, dadurch gekennzeichnet, daß man n-Butyllithium als lithiumorganische Verbindung einsetzt.

18. Verfahren nach Anspruch 1, dadurch gekennzeichnet, daß man die Umsetzung mit dem Wittig-Reagenz in Gegenwart eines Lösungsmittels stattfinden läßt.

19. Verfahren nach Anspruch 18, dadurch gekennzeichnet, daß man als Lösungsmittel einen Ether oder ein Sulfoxid einsetzt.

20. Verfahren nach Anspruch 19, dadurch gekennzeichnet, daß man als Lösungsmittel Tetrahydrofuran einsetzt.

21. Verfahren nach Anspruch 1, dadurch gekennzeichnet, daß man in Teilschritt 4 die Abspaltung der Schutzgruppe in Gegenwart eines Reaktionsmediums durchführt.

22. Verfahren nach Anspruch 21, dadurch gekennzeichnet, daß als Reaktionsmedium eine organische Säure oder ein Alkohol eingesetzt werden.

23. Verfahren nach Anspruch 22, dadurch gekennzeichnet, daß man als Reaktionsmedium Ethylenglykol einsetzt.
